# EUROPEAN PATENT SPECIFICATION

(11) **EP 1 206 926 B1**
(45) Date of publication and mention of the grant of the patent: **29.07.2009**
(21) Application number: 01127048.5
(22) Date of filing: 14.11.2001
(51) Int. Cl.: A61F 13/47, A61F 13/15

(54) **A sanitary napkin having regions of varying flexibility**
Damenbinde mit einzelnen Abschnitten unterschiedlicher Flexibilität
Serviette hygiénique avec des régions discrètes de fléxibilité variable

(30) Priority: 16.11.2000 US 714557
(43) Date of publication of application: 22.05.2002
(73) Proprietor: McNEIL-PPC, Inc., Skillman, NJ 08858 (US)
(72) Inventor: Hamilton, Alexandre, Montreal, Quebec, H2H 1M5 (CA); Pelley, Ken, Hopewell, NJ 08525 (US); Rosenfeld, Leonard G., East Windsor, NJ 08520 (US); Patel, Smith R., Edison, NJ 08820 (US); Mavinkurve, Pramod S., Princeton, NJ 08540 (US)
(74) Representative: Metten, Karl-Heinz

(56) References cited:
- EP-A- 0 293 208
- EP-A- 1 066 810
- EP-A- 1 077 051
- EP-A- 1 077 052
- EP-A- 1 078 617
- WO-A-93/21879
- WO-A-97/01996
- US-A- 5 575 786

## Description

### Field of the Invention

The present invention relates generally to sanitary napkins, which are thin, highly absorbent, and yet reasonably flexible.

### Background of the Invention

Sanitary absorbent articles find wide and varied use in absorbing and trapping body fluids and maintaining body surfaces in a state of dryness and comfort. The development of materials having a high liquid absorption capacity per unit volume has allowed the required overall thickness of sanitary absorbent articles to be reduced providing products which are less obtrusive to wear. Such articles find use, for example in feminine protection devices such as sanitary absorbent napkins. Thin sanitary napkins are generally constructed of multiple layers of material each having a particular function, as for example disclosed in U.S. Patent Number 5,575,786 to T.W. Osborne III. The sanitary napkin disclosed in this document includes a top sheet which is placed nearest the body surface of the wearer, an acquisition or transfer sheet with a relatively open structure having a relatively high void volume for accepting fluid and transporting fluid to an absorbent core which serves as the main dispository for liquid absorbed by the napkin. The napkin also has a barrier sheet which is impervious to liquid absorbed into the absorbent core and serves as a protective barrier between the absorbent core material and the wearer's clothing. The absorbent core has a high liquid absorption capacity relative to the top and transfer sheets and can be made from materials such as wood pulp, creped cellulose wadding, absorbent foams and sponges, polymeric fibres and polymeric gelling agents. The thickness of the napkin disclosed in U.S. Patent Number 5,575,786 is preferably less than 2.5 mm.

A problem which confronts designers of sanitary absorbent napkins is their ability to retain the absorbed liquid when subjected to mechanical loads as would be applied by the wearer in use. When subjected to such loading, liquid can leak from the absorbent core and rewet the layers above through which liquid was originally passed to the absorbent core. As the transfer and cover layers are made from materials with little absorption capacity, the liquid expelled from the absorbent core will tend to reside next to the body surface of the wearer resulting in discomfort and possible staining of the wearers garments. Typically in the prior art, this problem was addressed by utilizing materials of construction of the thin sanitary absorbent article, which improved its ability to retain liquids under load.

However, the material which provides adequate capacity In addressing this absorbency issue, is frequently too stiff for the user's comfort. The present invention attains improved user comfort by physically modifying one or more regions of a relatively stiff absorbent core to thereby improve flexibility in strategic areas of the napkin.

According to the present invention there is provided a sanitary napkin adapted to be worn in the crotch portion of an undergarment according to the features of claim 1.

### BRIEF DESCRIPTION OF THE DRAWINGS

Examples of embodiments of the present invention will now be described with reference to the drawings, in which:
Figure 1 is a top elevational view of a sanitary napkin not in accordance with an embodiment of the present invention, the cover layer of the sanitary napkin being partly removed to show the absorbent system;
Figure 2 is a perspective view of the sanitary napkin of Figure 1, depicted in a position attained when the sanitary napkin is placed in the undergarment of a wearer;
Figure 3 is a bottom plan view of the sanitary napkin shown in Figure 1;
Figure 4 is a cross-sectional view taken along the longitudinal centerline of the sanitary napkin shown in Figure 3;
Figure 5 is a schematic illustration of means for air-laying absorbent material for making an example of an absorbent core of the sanitary napkin according to an embodiment of the present invention, using four air-laying heads followed by means for compacting the air-layered material;
Figures 6a and 6b show three and four layer embodiments respectively of an absorbent core that can be used in the sanitary napkin of an embodiment of invention;
Figure 7a shows a top view of an embodiment of the invention in which the adhesive layer is discontinued in the region of the cuts in the absorbent region;
Figure 7b depicts a cross sectional view of the sanitary napkin shown in Figure 7a; and
Figures 8-11 each shows an alternative embodiment of the sanitary napkin of the present invention.

### DESCRIPTION OF THE PREFERRED EMBODIMENTS

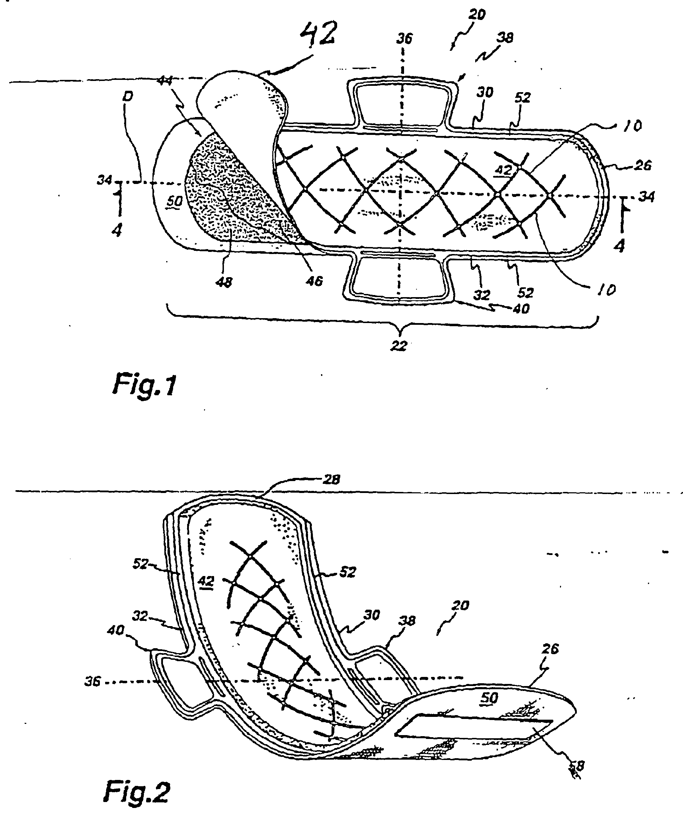

Referring to Figures 1 and 2, there is shown an embodiment of a feminine sanitary napkin 20.

The sanitary napkin 20 has a main body 22 with a first transverse side 26 defining a front portion thereof and a second transverse side 28 defining a rear portion thereof and defining therebetween a length. Each of these sides is preferably arcuate. The main body also has two longitudinal sides, namely a longitudinal side 30 and a longitudinal side 32. The main body has a width defined by the distance between longitudinal side 30 and longitudinal side 32. The sanitary napkin 20 has a thickness not exceeding about 5 mm. Preferably, the thickness is less than 3.5 mm, more preferably less than 3 mm, and most preferably, it is of about 2.8 mm.

The sanitary napkin 20 has a longitudinal centerline 34 that is an imaginary line bisecting the sanitary napkin 20 in two substantially identical halves. The main body 22 also has an imaginary transverse line 36 perpendicular to the longitudinal centerline 34.

Projecting laterally outward from each of the longitudinal sides 30, 32 is a flap 38, 40 (respectively). The flaps 38, 40 are in the shape of an isosceles trapezoid with the top adjoining the longitudinal side and the base at the distal end.

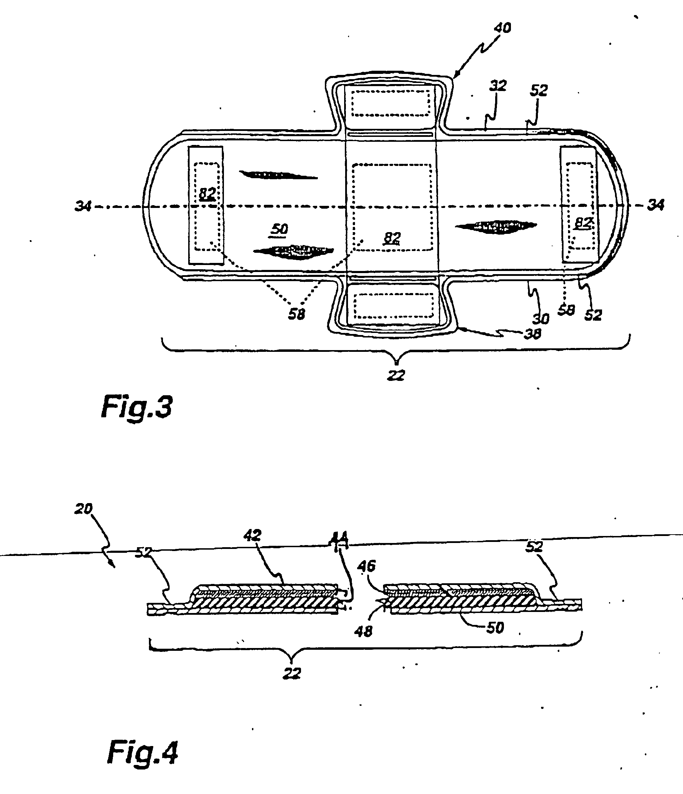

As depicted in Figure 4, the main body 22 is of a laminate construction and comprises a fibrous fluid-permeable body facing cover layer 42, an absorbent system 44, and a fluid-impervious barrier layer 50. The absorbent system is formed as a laminate of two or more layers of absorbent material. The absorbent system of a sanitary napkin of the invention comprises two component layers, namely a first absorbent layer 46 (commonly known as "transfer layer") and a second absorbent layer 48 (commonly known as "absorbent core"). Each of these layers is described in hereinbelow.

### Main Body-Cover Layer

The cover layer 42 may be formed from any flexible, liquid permeable material that is non-irritating to a user. Suitable liquid permeable materials include, but are not limited to woven fabrics, non-woven fabrics, apertured plastic films, and the like. The cover layer 42 is preferably a relatively low density, bulky, high-loft non-woven web material. The cover layer 42 may be composed of only one type of fiber, such as polyester or polypropylene or it may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. An example is the non-woven cover layer of sanitary napkins sold by Johnson & Johnson Inc. of Montreal, Canada under the trademark Stayfree Ultra-Thin Cottony Dry Cover.

Bi-component fibers may be made up of a polyester layer and a polyethylene sheath. The use of appropriate bi-component materials results in a fusible non-woven fabric. Examples of such fusible fabrics are described in U.S. Patent 4,555,446 issued November 50, 1985 to Mays. Using a fusible fabric increases the ease with which the cover layer may be mounted to the adjacent first absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Advantageously, the fibers which make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polyester or bi-component fibers, the cover layer 42 may be treated with a surfactant to impart the desired degree of wettability. An alternative embodiment would be that the cover layer 42 is composed of an apertured film.

The cover layer 42 may be affixed, e.g., by embossing to the remainder of the absorbent system 44 by affixing the cover to the underlying layer in order to assist fluid transport from the cover to the absorbent system. Such affixation may be effected locally, at a plurality of sites or over the entire contact surface of the cover layer 42 absorbent system 44. Exemplary means of affixing the cover layer to the absorbent system 44 are adhesion and fusion.

### Main Body - Absorbent System -First Absorbent Layer

Adjacent to the cover layer 42 on its inner side and bonded to the cover layer 42 is a first absorbent layer 46 that forms part of the absorbent system 44. The first absorbent layer 46 provides the means of receiving body fluid from the cover layer 42 and holding it until an underlying second absorbent layer has an opportunity to absorb the fluid, and therefore acts as a fluid transfer or acquisition layer.

The first absorbent layer 46 is, preferably, more dense than and has a larger proportion of smaller pores than the cover layer 42. These attributes allow the first absorbent layer 46 to contain body fluid and hold it away from the outer side of the cover layer 42, thereby preventing the fluid from re-wetting the cover layer 42 and its surface. However, the first absorbent layer 46 is, preferably, not so dense as to prevent the passage of the fluid through the layer 46 into the underlying second absorbent layer 48.

The first absorbent layer 46 may be composed of fibrous materials, such as wood pulp, polyester, rayon, or the like, or combinations thereof. Alternatively, the first absorbent layer 46 may be composed of non-fibrous materials such as flexible foam. In a preferred embodiment, the first absorbent layer 46 is composed of fibrous materials and may include thermoplastic fibers for the purpose of stabilizing the layer and maintaining its structural integrity. The first absorbent layer 46 may be treated with surfactant on one or both sides in order to increase its wettability, although generally the first absorbent layer 46 is relatively hydrophilic and may not require treatment. The first absorbent layer 46 is preferably bonded on both sides to the adjacent layers, i.e. the cover layer 42 and an underlying second absorbent layer 48.

Materials particularly suitable for use in the first absorbent layer 46 have a density in the range of about 0.04 to 0.05 g/cc, a basis weight in the range from about 80 to 110 g/m² and a thickness in the range of about 2 to 3 mm and in particular a thickness of 2.6 mm. Examples of suitable materials for the first absorbent layer are through air bonded pulp sold by Buckeye of Memphis, Tennessee, under the designation VIZORB 3008, which has a basis weight of 110 g/m² and VIZORB 3010, which has a basis weight of 90 g/m².

### Main Body -- Absorbent System-Second Absorbent Layer

Immediately adjacent to and bonded to the first absorbent layer 46 is the second absorbent layer 48.

As noted above, the absorbent system comprises both the first and second absorbent layers. In one embodiment, the first absorbent layer 46 has a width that is at least about the same as the width of the second absorbent layer 48. In another embodiment, the first absorbent layer 46 has a width that exceeds the width of the second absorbent layer 48. In either of these embodiments, the "width" of the absorbent system is defined to be the smaller of the widths of the first and second absorbent layers and it refers to the maximum measurement across that layer along a line perpendicular to the longitudinal centerline.

In the preferred embodiment, the width of the absorbent system exceeds 45 mm. In an alternative embodiment the minimum width of the absorbent system is 64 mm.

In one embodiment, the second absorbent layer 48 is a blend or mixture of cellulosic fibers and superabsorbent disposed in and amongst fibers of that pulp.

In a specific example, the second absorbent layer 48 is a material containing from about 40 weight percent to about 95 weight percent cellulosic fibers and, more specifically from about 60 to about 80 weight percent cellulosic fibers. Such a material may contain from about 5 weight percent to about 60 weight percent SAP (superabsorbent polymers), preferably form about 20 to about 55 weight SAP, and even more preferably from about 30 to 45 weight percent SAP, and most preferably about 40 weight percent SAP. The material has a water content of less than about 10 weight percent. As used herein, the phrase "weight percent" means weight of substance per weight of final material. By way of example, 10 weight percent SAP means 10 g/m² SAP per 100g/m² basis weight of the material.

Cellulosic fibers that can be used in the second absorbent layer 48 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material.

The second absorbent layer 48 can contain any superabsorbent polymer (SAP), which SAPs are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA60N Type II*, the product offered by Stockhausen, Inc. of Greensboro, North Carolina, under the designation of 7440, and the product offered by Chemdal International, Inc. of Palatine, Illinois, under the designation of 2100A*.

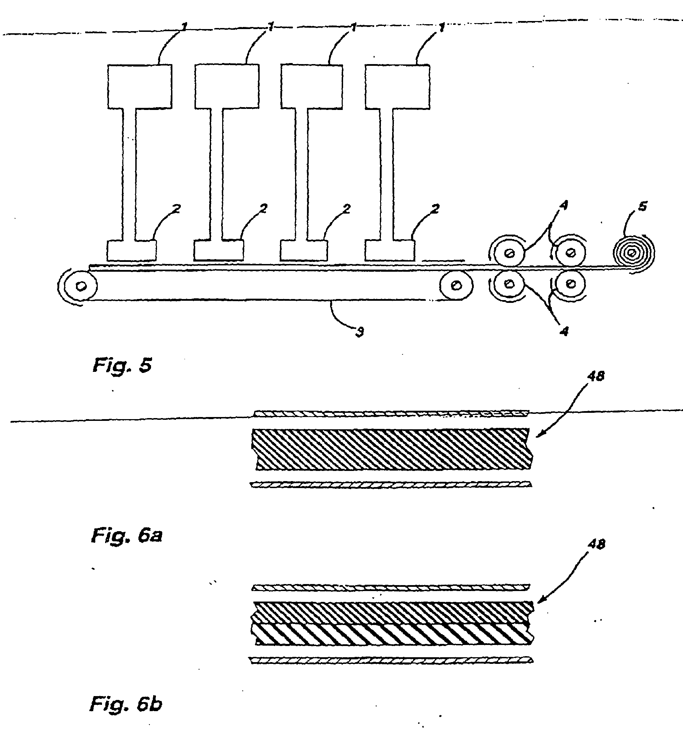

The second absorbent layer 48 can be manufactured by using air-laying means. In accordance with Figure 5, cellulosic fibers (e.g., pulp) are processed using a hammer mill to individualize the fibers. The individualized fibers are blended with SAP granules in a blending system 1 and pneumatically conveyed into a series of forming heads 2. The blending and distribution of fibers and SAP granules can be controlled separately for each forming head. Controlled air circulation and winged agitators in each chamber produce uniform mixture and distribution of pulp and SAP. The SAP can be thoroughly and homogeneously blended throughout the material or contained only in specific strata by distributing it to selected forming heads. Fibers (and SAP) from each forming chamber are deposited by vacuum onto a forming wire 3 thus forming a layered absorbent web. The web is subsequently compressed using calendars 4 to achieve desirable density. The densified web is wound into a roll 5 using conventional winding equipment. The forming wire 3 can be covered with tissue paper to reduce the loss of material. The tissue paper layer can be removed prior to calendering or incorporated into the formed material. In a possible variant, the first absorbent layer 46 can be formed integrally with the second absorbent layer 48 to provide a unitized absorbent system 44. This can be achieved by providing the apparatus depicted in Figure 5 with an additional forming head (not shown in the drawings) to deposit on the second absorbent layer 48, by air laying and prior to calendering, a layer of material to form the first absorbent layer 46.

The second absorbent layer 48 of the present invention is of high density and in a specific example has a density of greater than about 0.25 g/cc. Specifically, the second absorbent layer 48 may have a density in the range of from about 0.25 g/cc to about 0.50 g/cc. More specifically, the density is from about 0.25 g/cc to about 0.40 g/cc and, even more specifically from about 0.25 g/cc to about 0.35 g/cc.

Air-laid absorbents are typically produced with a low density. To achieve higher density levels, such as the examples of the second absorbent layer 48 given above, the air-laid material is compacted using calenders as shown in Figure 5. Compaction is accomplished using means well known in the art. Typically such compaction is carried out at a temperature of about 100 degrees C and a load of about 130 Newtons per millimeter. The upper compaction roll is typically made of steel while the lower compaction roll is a flexroll having a hardness of about 85 SH D. It is preferred that both the upper and lower compaction rolls be smooth, although the upper roll can be engraved.

The second absorbent layer 48 can be prepared over a wide range of basis weights. The second absorbent layer 48 can have a basis weight in the range of from about 100 g/m²to about 700 g/m². In a specific example, the basis weight ranges from about 150 g/m² to about 400 g/m². Preferably the basis weight ranges from about 200 g/m² to about 350 g/m² and, more preferably, to about 250 g/m².

The first absorbent layer 46 functions to rapidly absorb and retain fluid which is then more slowly absorbed by the second absorbent layer 48. The first absorbent layer having a relatively open pore structure readily absorbs and disperses liquid laterally within its bulk and readily transfers the liquid to the receiving surface of the absorbent core. In turn, the absorbent core having a relatively smaller pore structure than the first absorbent layer has good capillarity which efficiently draws liquid into its bulk from the first absorbent layer. Once the liquid has been absorbed into superabsorbent polymer, the liquid cannot be subsequently released by applying pressure. Therefore, the liquid absorbed into the superabsorbent material becomes entrapped. At the same time, the strength with which second absorbent layer intakes liquid from the first absorbent layer helps to reduce the proportion of liquid held in the first absorbent layer, thereby reducing the amount of liquid that returns to the cover layer when the napkin is subjected to mechanical loading. Furthermore, the first absorbent layer has a relatively high capillarity so that any concentration of liquid in the first absorbent layer resulting from mechanical loading can be redistributed within the material to lower concentrations, again reducing the amount of liquid which can return to the cover layer.

In a specific embodiment, the second absorbent layer 48 contains in the range from about 30 to 40 weight percent superabsorbent material, has a basis weight in the range from about 200 to 400 g/m² and a density in the range from about 0.2 to 0.5 g/cc. More specifically, the density is from about 0.25 g/cc to about 0.45 g/c and, even more specifically about 0.3 g/cc.

The second absorbent layer 48 can be formed as three or four lamina or strata. Those strata include a bottom layer, one or two middle layers and a top layer. Specific examples of three and four layer material are set forth below. The SAP can be included in any or all of the layers. The concentration (weight percent) of SAP in each layer can vary as can the nature of the particular SAP.

Even where prepared as from multiple layers, the final thickness of the formed second absorbent layer 48 is low. The thickness can vary from less than about 0.5 mm to about 2.5 mm. In a specific example, the thickness is from less than about 0.5 mm 1.5 mm.

In one embodiment, the cellulosic fiber for use in the second absorbent layer 48 is wood pulp. There are certain characteristics of wood pulp that make it particularly suitable for use. Cellulose in most wood pulps has a crystalline form known as Cellulose I which can be converted to a form known as Cellulose II. In the second absorbent layer 48, wood pulp with a substantial portion of the cellulose as Cellulose II could be used. Similarly, pulps having an increased fiber curl value are advantageous. Finally, pulps having reduced levels of hemicellulose are preferred. Means for treating pulps so as to optimize these characteristics are well known in the art. By way of example, treating wood pulp with liquid ammonia is known to convert cellulose to the Cellulose II structure and to increase the fiber curl value. Flash drying is known to increase the fiber curl value of pulp. Cold caustic treatment of pulp decreases hemicellulose content, increases fiber curl and converts cellulose to the Cellulose II form. Thus it could be advantageous that the cellulosic fibers used to produce the material of this invention contain at least a portion of cold caustic treated pulp.

Briefly, a caustic treatment is typically carried out at a temperature less than about 60 degree C., but preferably at a temperature less than 50 degree C., and more preferably at a temperature between about 10 degree C. to 40 degree C. A preferred alkali metal salt solution is a sodium hydroxide solution newly made up or as a solution by-product in a pulp or paper mill operation, e.g., hemicaustic white liquor, oxidized white liquor and the like. Other alkali metal salts such as ammonium hydroxide and potassium hydroxide and the like can be employed. However, from a cost standpoint, the preferable salt is sodium hydroxide. The concentration of alkali metal salts is typically in a range from about 2 to about 25 weight percent of the solution, and preferably from about 6 to about 18 weight percent. Pulps for high rate, fast absorbing applications are preferably treated with alkali metal salt concentrations from about 10 to about 18 weight percent.

For further details on the structure and the method of construction of the second absorbent layer 48 the reader is invited to refer to the US patent 5,866,242 granted on February 2, 1999 to Tan et al.

### Main Body-Barrier Layer

Underlying the absorbent system 44 is a barrier layer 50 comprising liquid-impervious film material so as to prevent liquid that is entrapped in the absorbent system 44 from egressing the sanitary napkin and staining the wearer's undergarment. The barrier layer 50 is preferably made of polymeric film, although it may be made of liquid-impervious air-permeable material such as repellent-treated, non-woven or microporous films or foams.

The cover layer 42 and the barrier layer 50 are joined along their marginal portions so as to form an enclosure or flange seal that forms a unitary absorbent product and maintains the absorbent system 44 captive. The joint may be made by means of adhesives, heat-bonding, ultrasonic bonding, radio frequency sealing, mechanical crimping, and the like and combinations thereof. The peripheral seal line is shown in Figure 1 by the reference numeral 52.

### Flaps

The flaps 38 and 40 are preferably made as integral extensions of the cover layer 42 and the barrier layer 50. These integral extensions are joined to one another along their marginal seal portions by adhesives, heat-bonding, ultrasonic bonding, radio frequency sealing, mechanical crimping, and the like and combinations thereof. Most preferably, such joining is made at the same time the cover layer 42 and the barrier layer 50 are bonded to one another to enclose the absorbent system 44. Alternatively, the flaps may include absorbent material between the cover layer and the barrier layer extensions. Such absorbent material may be an extension of the first absorbent layer 46, the second absorbent layer 48 or both.

### Adhesive system

Referring to Figures 2 and 3, in order to enhance the stability of the sanitary napkin, the garment facing surface of the barrier layer is provided with an undergarment attachment or positioning adhesive material 58, typically hot-melt adhesive material capable of establishing a temporary bond with the undergarment material. A suitable material is the composition designated HL-1491 XZP commercially available from H.B. Fuller Canada, Toronto, Ontario, Canada. The positioning adhesive 58 may be applied to the garment-facing surface of the barrier layer 50 in various patterns, including complete adhesive coverage, parallel longitudinal lines, a line of adhesive following the perimeter of the structure, transverse lines of adhesive or the like.

Standard release paper 82 (shown only in Figure 3) covers the positioning adhesive 58 before the napkin is used to prevent the unwanted adherence of the napkin to itself or foreign objects. The release paper is of conventional construction (e.g. silicone coated wet-laid Kraft wood pulp) and suitable papers are available from Tekkote Corporation (Leonia, New Jersey, USA), and bear the designation FRASER 30#/61629.

### CHANNEL FORMATIONS

In a preferred embodiment, the sanitary napkin is provided with at least one and preferably more than one channel formation arranged to direct liquid along the channel (or channels) for subsequent absorption into the first absorbent layer. The channels are formed by embossing and densifying one or more regions of the napkin. The inventors have found that the provision of channels contributes significantly in reducing the rewet potential. Preferably, the napkin has a plurality of elongate channels formed therein, which are spaced apart from each other and configured to channel liquid laterally across the body-facing surface of the napkin or near body facing surface portion thereof, away from the region of initial deposition.

The provision of one or more channels adjacent the cover layer enables liquid to be transported rapidly over the napkin so that different regions of the first absorbent layer act effectively to absorb the liquid in parallel. This helps to ensure that liquid is presented to a larger portion of the surface area of the second absorbent layer to increase the effectiveness of the second absorbent layer in drawing liquid from the first absorbent layer.

The napkin may be provided with a single channel or multiple channels, for example running along or parallel to the longitudinal axis along the length of the napkin, obliquely of the longitudinal axis, for example from one side of the napkin to the other or substantially perpendicular to the longitudinal axis. The channel(s) may have any shape which may be selected according to the particular application, for example the channels may be linear, arcuate or have a serpentine configuration or a mixture of these as well as other shapes, including a spiral and zig-zag patterns.

In one embodiment, the napkin has a plurality of discrete channel formations which are spaced apart and intersect one another. An example of such an embodiment is shown in Figure 1. Referring to Figure 1, the napkin 20 is provided with a plurality of arcuate channels 10 which extend generally obliquely of the longitudinal centre line 34 from one half of the napkin surface formed by the centre line 34 to the other half. This design efficiently conducts liquid simultaneously along the length and across the width of the napkin. The channel formation may be formed in the cover layer and/or in the first absorbent layer. The channels may be formed advantageously by applying localised pressure to the material as for example is used in embossing. The applied pressure results in densifying the material which defines the floor of the channel rendering it less pervious to liquid and so extending the distance over which the liquid can travel before absorption. The first absorbent (transfer) layer is preferably relatively thick in comparison with the other layers of the napkin which enables relatively deep channels to be formed. Advantageously, portions of the transfer layer laterally adjacent to the channel remain relatively thick and retains their original, relatively open pore structure allowing liquid to be efficiently drawn from the channel. Advantageously, the transfer layer comprises thermoplastic fibres. The provision of thermoplastic fibres assists in the formation of a stable and permanent channel when the thermoplastic fibres and subjected to heat. When heat is applied, the thermoplastic fibres tend to fuse together to form a more rigid structure so that the original form of the channels is maintained during use and over time. Conveniently, the application of heat may be incorporated with the embossing process.

### Central Absorbent Zone

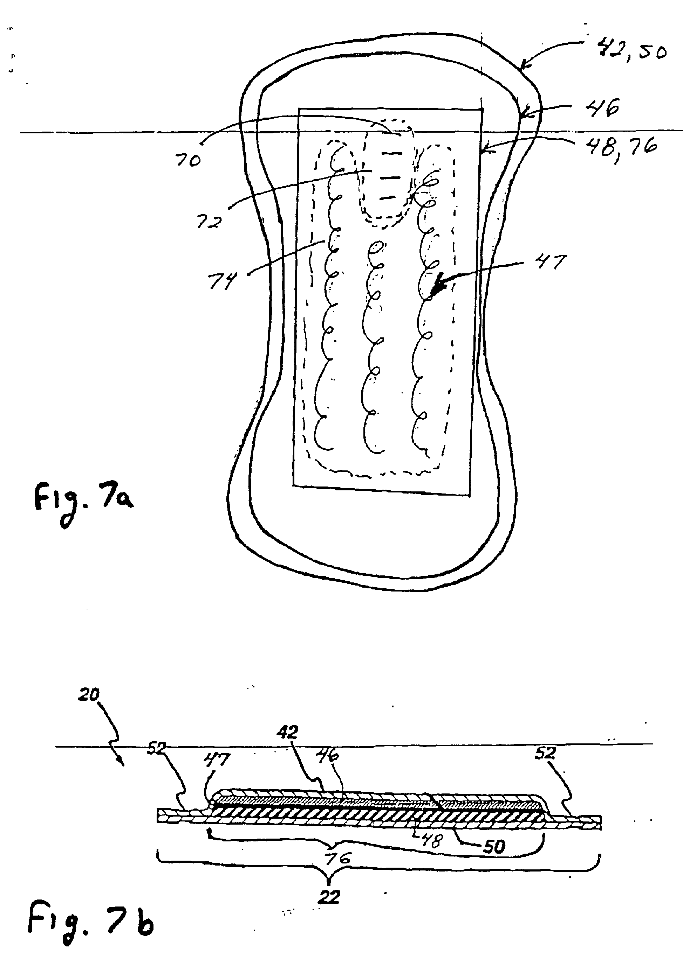

The section of the napkin comprising in vertical registration the cover, absorbent system and barrier and wherein there is substantially uniform composition of absorbent material, is referred to as the central absorbent zone. Figures 7a and 7b illustrate an embodiment of a sanitary napkin in which this central absorbent zone 76 is depicted. The stiffness of this zone plays a key role in the performance of the product. If the central absorbent zone is uniformly too stiff it will not deliver the comfort which is desirable in a thin product. If this zone is uniformly too flexible it will result in bunching of the product. The central absorbent zone requires that the absorbent system include the second absorbent layer 48.

An important aspect of the current invention is that one or more regions of the central absorbent zone will have adequate stiffness to avoid extensive bunching. One or more other regions of the central absorbent zone will have a somewhat lower stiffness to insure that the comfort properties of the product are maintained. The napkin is designed to deform in a controlled manner in these regions. The lower stiffness is obtained in a manner that maintains substantially uniform composition of absorbent materials in the central absorbent zone 76 to thereby maintain good absorbency properties. As used herein the terminology "substantially uniform composition of absorbent materials" means that the concentration of absorbent materials is substantially uniform. Thus, the variations in stiffness within the central absorbent zone are not achieved by the addition of or removal of absorbent or other material but in fact are achieved by a mechanical modification of the absorbent material itself.

As an illustrative example, Figure 7a depicts such a first region 74 of higher stiffness and a second region 72 of reduced stiffness. As used herein, the terminology "region" refers to an area of at least 12.7 mm by 25.4 mm. In particular, the first region 74 is that area the central absorbent zone which remain unmodified, while the second region 72 is that area in which cuts 70 have been added. These cuts are into the second absorbent layer, the first absorbent layer or both. Figures 7a and 7b also depict an adhesive layer 47 for bonding the first absorbent layer 46 to the second absorbent layer 48. As illustrated in Figure 7a, there is a void in the adhesive layer in the second region 72.

Preferably the first region (i.e., having higher stiffness) of the central absorbent zone will have a stiffness greater than 300 grams as measured by the peak bending stiffness. By the Gurley stiffness method (described in detail below) it will have a stiffness measurement greater than about 350 mg. The second region of the central absorbent zone will have stiffness values of at least about 10% less than first region measurement in either of these tests.

According to the present invention the main absorbent materials will substantially have uniform absorbent composition in the central absorbent zone 76 to assure uniform absorbency properties throughout the central absorbent zone. The lower stiffness values will be obtained by means other than modified absorbent composition. Selective use of adhesives between absorbent layers is one such method. That is, those areas where the layers are adhesively bonded will be stiffer than those areas where no adhesive or bonding is present between layers.

Other means may be by mechanically modifying either all of the layers in a section of the central absorbent zone or any individual material in a section of the central absorbent zone. Methods of modification may include adding cuts, slits perforations or tenderizing as discussed U.S. Patent Number 4,605,402 to M. Iskra and U.S. Patent Number 5,466,232 to S. Cadieux et al.

Adding selective cuts or perforations is a preferred approach and can be done with standard rotating process equipment. This approach will typically add cuts to selective regions of one or both absorbent layers. The cuts would not typically be added to either the barrier or the cover for obvious fluid containment reasons. The cuts may be individual cuts or they may be multiple cuts in the region of each other to effect the stiffness of the product and provide regions where the product easily deforms under body stresses. The cuts will be more effective if there is no adhesive bonding the absorbent layers in the region of the cuts. Flexibility may also be provided by single or multiple cuts or slits or any variety of perforating, tenderizing or scoring.

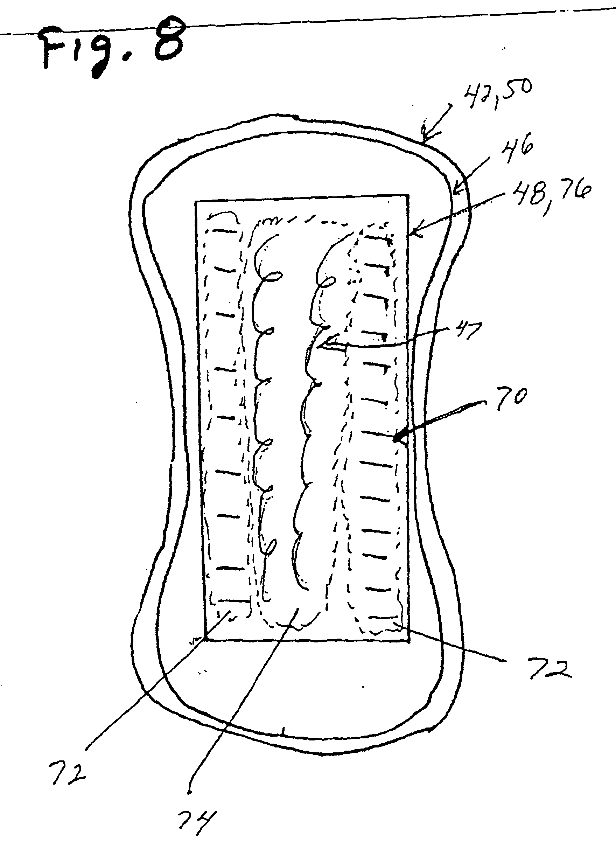

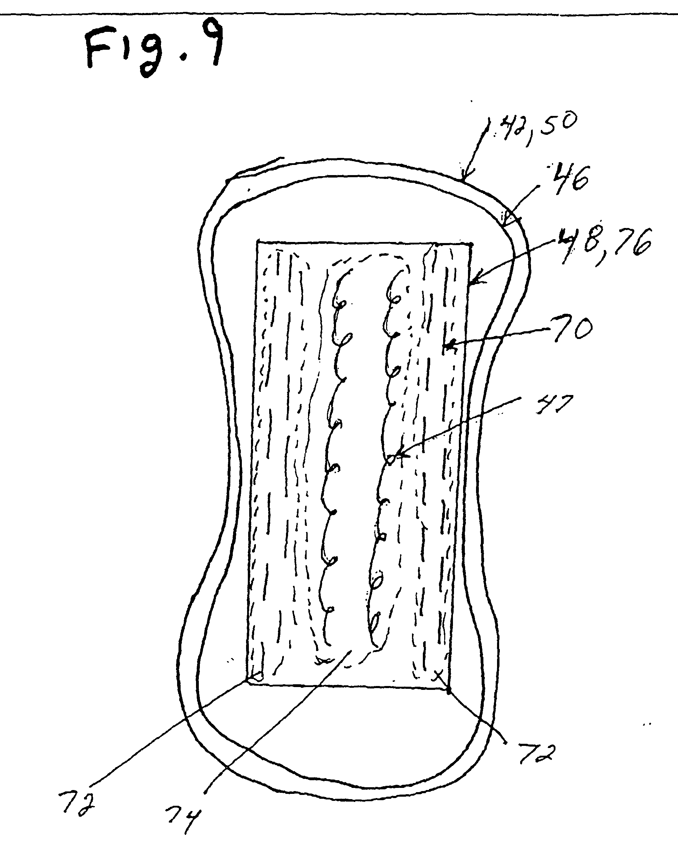

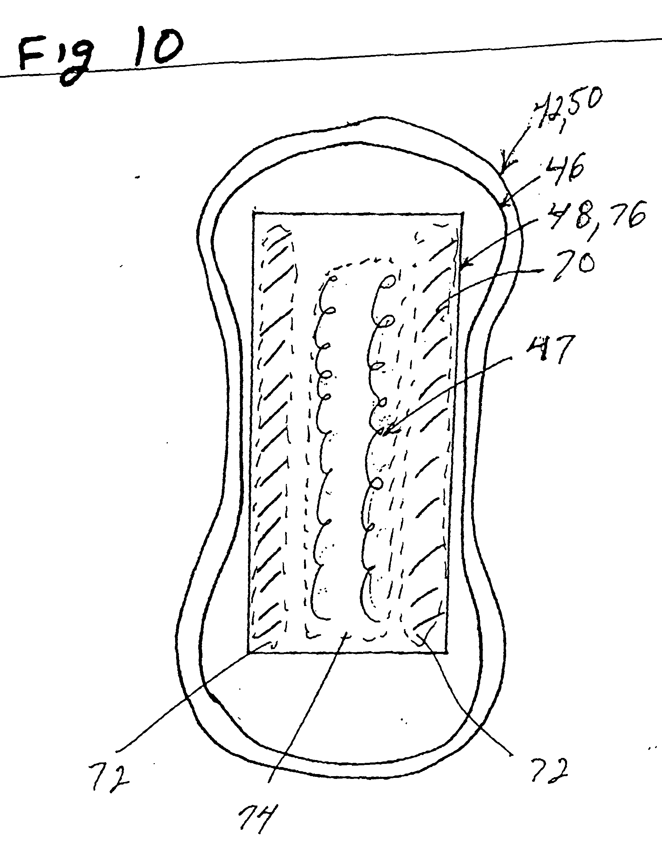

Figures 8-10 each show an alternative embodiment of a sanitary napkin having a central absorbent zone comprising a centrally located, longitudinally extending first region having a higher stiffness and a second region of reduced stiffness adjacent each longitudinal edge of the central absorbent zone. In particular, the first regions 74 are those areas of the central absorbent zone which remain unmodified, while the second regions 72 of decreased stiffness are those in which a plurality of cuts or slits 70 have been added. These cuts are into the second absorbent layer, the first absorbent layer, or both. Each of these figures depicts an adhesive layer 47 for bonding the first absorbent layer 46 to the second absorbent layer 48. As illustrated in each of the figures, there is a void in the adhesive layer in the second region of the central absorbent zone and thus the first and second absorbent layers are unaffixed in the second region. Similar voids of a bonding means are present in the reduced stiffness regions of alternative embodiments in which adhesives are not used as the bonding means between layers.

In one embodiment of the present invention, the width of the second region 72, measured in a direction perpendicular to the longitudinal centerline 34, exceeds 37.5 mm and the length of this region, measured in a direction parallel to the longitudinal centerline 34, also exceeds 37.5 mm. In an alternative embodiment, the corresponding minimum width dimension is 12.7 mm while the length dimension exceeds 25.4 mm.

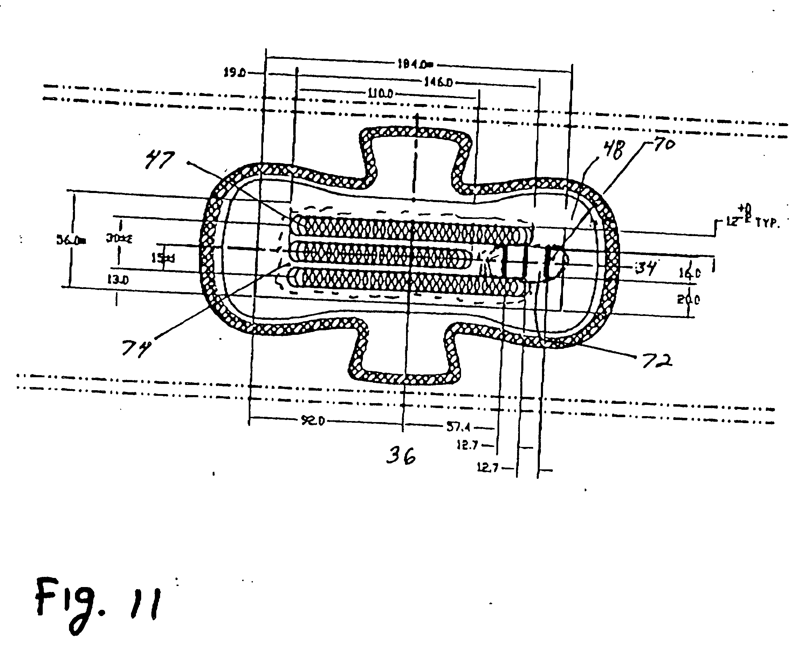

Figure 11 illustrates a preferred embodiment of the invention in which three transverse cuts 70 have been added. Each of these cuts is 16.0 mm wide and each is perpendicular to and bisected by the longitudinal centerline 34. These cuts are performed on the second absorbent layer 48 and are complete cuts through the entire thickness of that layer (as opposed to merely scoring the layer). Further, Figure 11 depicts the specific positioning of these cuts relative to the longitudinal centerline 34 and the imaginary transverse line 36. Still further, Figure 11 depicts specific dimensions of the adhesive layer 47 and thereby illustrates the absence of adhesive in the reduced stiffness region 72 in which the cuts are located. As illustrated in the embodiment of Figure 11 the length of the reduced stiffness region exceeds 25.4 mm while the width exceeds 16.0 mm.

### Method of manufacture

The above-described embodiment of the sanitary napkin 20 is fabricated in a conventional manner in accordance with conventional techniques. Specifically, a laminate structure, sometimes referred to in the art as a web, is created. This laminate structure comprises an expanse of the materials from which the napkin will be created. In other words, the laminate structure comprises the following layers of material in a top-to-bottom order: an expanse of cover layer material; an expanse of first absorbent layer material; an expanse of second absorbent layer material (manufactured as described above); and finally an expanse of barrier layer. Some of the materials are necessarily not continuous within the laminate structure, and where such is the case, they are positioned precisely, one with respect to another, in the relationship they will occupy in the final products. The cover layer material and the barrier layer material are then bonded together by applying pressure in the appropriate positions, and what will become the peripheral seal is created. (The seal may also be made by means of heat-bonding, ultrasonic bonding, radio frequency sealing, mechanical crimping, and the like and combinations thereof.) The sealed structure is then severed by conventional means (i.e. die-cutting, fluid-jet cutting, or by laser) from the web to create a discrete article.

As mentioned above, one or more channels may be formed adjacent the body facing surface of the napkin, and the channel(s) may be formed for example by embossing. The channel(s) may be formed by other techniques, including cutting, excavating, etching, molding and cauterizing, as well as other methods known to those skilled in the art. If embossing is used, the method may involve passing the sanitary napkin between a pair of rollers, in which one of the rollers includes projections configured to the desired embossing pattern. The projections compress and density the material locally and may be applied to the cover layer, the absorbent system (particularly, the first absorbent layer) or a combination of the two. The degree of pressure applied during the embossing operation depending upon the type of material and its physical integrity. Finding the optimal process conditions in accordance with the specific application is within the scope of a person skilled in the art. In general, the embossing pressure should be selected to sufficiently densify the material locally to form the channels but not too high so as to sever the material. As mentioned above, the material may also be heated and this may be done conveniently by heating the embossing rollers. Ultrasonic embossing may also be used for forming the channel(s).

Advantageously, embossing helps to hold the various layers of the sanitary napkin together and reduces the likelihood of the cover layer or the barrier layer separating from the adjacent layers or coming loose when the sanitary napkin is bent. Preferably, the napkin is embossed at regular intervals over the majority and preferably the entirety of its surface.

The positioning adhesive material is then applied to the barrier layer in the appropriate positions, and release paper is applied to cover the positioning adhesive. Alternatively, the positioning adhesive, or the positioning adhesive and the release paper may be applied to the web before the individual articles are severed therefrom.

### Procedure for Measuring the Thickness of a Sanitary Article

As indicated earlier, the sanitary napkin 20 has a thickness of about 5 mm or less. The apparatus required to measure the thickness of the sanitary napkin is a footed dial (thickness) gauge, available from Ames, with a foot having a 1 1/8" diameter and a stand, 2 oz. deadweight accurate to 0.001". A digital type apparatus is preferred. If the sanitary napkin sample is individually folded and wrapped, the sample is unwrapped and carefully flattened by hand. The release paper is removed from the sample and it is repositioned back gently across the positioning adhesive lines so as not to compress the sample, ensuring that the release paper lies flat across the sample. Flaps (if any) are folded back under the sample, prior to taking the thickness reading in the center of the sample.

The foot of the gauge is raised and the sample is placed on the anvil such that the foot of the gauge is approximately centered the sample (or in the location of interest on the sample of interest). When lowering the foot, care must be taken to prevent the foot dropping onto the sample or undue force being applied. After the foot is lowered, the sample and read out are allowed to stabilize for approximately 5 seconds. The thickness reading is then taken.

### Procedure for Measuring Peak Bending Stiffness

Peak bending stiffness is determined by a test that is modelled after the ASTM D 4032-82 CIRCULAR BEND PROCEDURE, the procedure being considerably modified and performed as follows. The CIRCULAR BEND PROCEDURE is a simultaneous multi-directional deformation of a material in which one face of a specimen becomes concave and the other face becomes convex. The CIRCULAR BEND PROCEDURE gives a force value related to flexural resistance, simultaneously averaging stiffness in all directions.

The apparatus necessary for the CIRCULAR BEND PROCEDURE is a modified Circular Bend Stiffness Tester, having the following parts:
1. A smooth-polished steel plate platform which is 102.0 mm by 102.0 by 6.35 mm having an 18.75 mm diameter orifice. The lap edge of the orifice should be at a 45 degree angle to a depth of 4.75 mm;
2. A plunger having an overall length of 72.2 mm, a diameter of 6.25 mm, a ball nose having a radius of 2.97 mm and a needlepoint extending 0.88 mm therefrom having a 0.33 mm base diameter and a point having a radius of less than 0.5 mm, the plunger being mounted concentric with the orifice and having equal clearance on all sides. Note that the needle-point is merely to prevent lateral movement of the test specimen during testing. Therefore, if the needlepoint significantly adversely affects the test specimen (for example, punctures an inflatable structure), than the needlepoint should not be used. The bottom of the plunger should be set well above the top of the orifice plate. From this position, the downward stroke of the ball nose is to the exact bottom of the plate orifice;
3. A force-measurement gauge and more specifically an Instron inverted compression load cell. The load cell has a load range of from about 0.0 to about 2000.0 g;
4. An actuator and more specifically the Instron Model No. 1122 having an inverted compression load cell. The Instron 1122 is made by the Instron Engineering Corporation, Canton, Mass.

Five representative sanitary napkins are used for the procedure. For each of these napkins one or more sections in the central absorbent zone are selected from its second region, where lower stiffness values are expected. That is, in these sections cuts or other modifications have been added. In addition, one or more sections in the central absorbent zone are selected from its first region areas wherein no modifications have been made and consequently a higher stiffness value is expected. A 37.5 mm by 37.5 mm sample is cut from each of these selected regions.

The test specimens should not be folded or bent by the test person, and the handling of specimens must be kept to a minimum and to the edges to avoid affecting flexural-resistance properties. From the four remaining sanitary napkins, an equal number "Y" of 37.5 mm by 37.5 mm specimens, identical to the specimens cut from the first napkin, are cut. Thus, the test person should have "Y" number of sets of five identical specimens.

The procedure for the CIRCULAR BEND PROCEDURE is as follows. The specimens are conditioned by leaving them in a room that is 21 degree Celsius plus or minus 0.1 degree Celsius and 50% plus or minus 2.0% relative humidity for a period of two hours. The test plate is leveled. The plunger speed is set at 50.0 cm per minute per full stroke length. A specimen is centered on the orifice platform below the plunger such that the cover layer 42 of the specimen is facing the plunger and the barrier layer 50 of the specimen is facing the platform. The indicator zero is checked and adjusted, if necessary. The plunger is actuated so that it presses the specimen in to the orifice until the bottom of the ball nose is coplanar with the bottom of the orifice plate. Touching the specimen during the testing should be avoided. The maximum force reading to the nearest gram is recorded. The above steps are repeated until all five of the identical specimens have been tested.

### Calculations

The peak force is recorded for each specimen when it is tested. For each tested region on the napkins, the average of the 5 peak forces is calculated and is equal to the peak bending stiffness for that region of the napkin. For the cases where multiple regions are tested, the maximum of the stiffer regions and the lowest of the more flexible regions are taken as representative of the product.

### Gurley Stiffness Measurement

Stiffness values can be measured by either the Peak Bending Stiffness described above or by the Gurley method. As the Gurley method uses a smaller sample size, it may be more appropriate when the regions of interest is smaller than the 37.5 mm square sample which is used for the Peak Bending Stiffness Method.

Gurley stiffness is one of many indices of stiffness. Gurley stiffness measures the bendability or flexibility of absorbent materials. The lower the Gurley stiffness value, the more flexible the material. The Gurley stiffness values are measured using a Gurley Stiffness Tester (Model No. 4171E), manufactured by Gurley Precision Instruments of Troy, N.Y. The instrument measures the externally applied moment required to produce a given deflection of a test strip of specific dimensions fixed at one end and having a concentrated load applied to the other end. The results are obtained in "Gurley Stiffness" values in units of milligrams.

Similar to the PBS method, one or more sections in the central absorbent zone are selected from its first region, where the highest stiffness values are expected. As before one or more sections are also selected from the second region of the central absorbent zone, where lower stiffness values are expected. These are areas where cuts or other modifications have been added.

From each chosen section on the five napkins, 12.7 by 25.4 mm samples are cut and tested by the standard Gurley stiffness testing procedures where a result in milligrams is reported by the testing device with the proper input parameters. Note that the test is performed with one of the 12.7 mm ends of the samples placed in the clamp and with the average values taken from deflecting the sample in both directions.

The test samples can be chosen with either the long or short dimensions of the samples in the longer direction of the product This decision is to be made based on in a manner to maximize the difference between the stiffest and most flexible positions. In the case where cuts are added to the absorbent core, the 12.7 mm direction will be parallel to the cuts.

The average values for each region are calculated. For the cases where multiple regions are tested, the maximum of the stiffer regions and the lowest of the more flexible regions are taken as representative of the product.

Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims and their equivalents.

## Claims

1. A sanitary napkin (20) having a thickness of less than 5 mm and comprising a central absorbent zone (76); the central absorbent zone (76) comprising a fluid-permeable, body-facing cover layer (42); a fluid impermeable, garment facing barrier layer (50), and an absorbent system (44); a concentration of absorbent materials being uniform in the central absorbent zone (76); the central absorbent zone (76) having a width of at least 45 mm; the central absorbent zone (76) further comprising a first region (74) and the second region (72) adjacent the first region (74), the first region (74) having a stiffness, and the second region (72) having a reduced stiffness that is less than 90% of the stiffness of the first region (74); the absorbent system (44) having a first (46) and a second absorbent layer (48); the second region (72) containing cuts either in the first (46) or in the second absorbent layer (48) or in both, **characterized in that** an adhesive layer (47) bonds the first absorbent layer (46) to the second absorbent layer (48), and that there is a void in the adhesive layer (47) in the second region (72) of the central absorbent zone (76) so that the first and the second absorbent layer (46, 48) are unaffixed in the second region (72).

2. The sanitary napkin (20) according to claim 1, wherein the central absorbent zone (76) comprises a centrally located, longitudinally extending first region (74) and a second region (72) adjacent each longitudinal edge of the central absorbent zone (76).

3. The sanitary napkin (20) as claimed in claim 1, wherein said stiffness of the first region (74) is greater than 300 grams as measured by the peak bending stiffness method.

4. The sanitary napkin (20) as claimed in claim 1, wherein said stiffness of the first region (74) is greater than 350 mg as measured by the Gurley stiffness method.

5. The sanitary napkin (20) as claimed in claim 3, wherein said second region (72) has a stiffness that is less than 80% of the stiffness of the first region (74).

6. The sanitary napkin (20) as claimed in claim 1, wherein said second region (72) has a stiffness that is less than 60% of the stiffness of the first region (74).

## Patentansprüche

1. Damenbinde (20) mit einer Dicke von weniger als 5 mm und umfassend eine zentrale absorbierende Zone (76), wobei die zentrale absorbierende Zone (76) eine flüssigkeitsdurchlässige, dem Körper zugewandte Decklage (42), eine flüssigkeitsundurchlässige, der Kleidung zugewandte Barrierelage (50) und ein absorbierendes System (44) umfasst; wobei die Konzentration an absorbierenden Materialien in der zentralen absorbierenden Zone (76) einheitlich ist; wobei die zentrale absorbierende Zone (76) eine Breite von mindestens 45 mm aufweist; wobei die zentrale absorbierende Zone (76) weiterhin einen ersten Abschnitt (74) und den zweiten Abschnitt (72) benachbart zu dem ersten Abschnitt (74) umfasst, wobei der erste Abschnitt (74) eine Steifigkeit aufweist, und der zweite Abschnitt (72) eine verringerte Steifigkeit aufweist, die geringer ist als 90 % der Steifigkeit des ersten Abschnitts (74); wobei das absorbierende System (44) eine erste (46) und eine zweite absorbierende Lage (48) aufweist; wobei der zweite Abschnitt (72) Einschnitte entweder in der ersten (46) oder in der zweiten absorbierenden Lage (48) oder in beiden aufweist, **dadurch gekennzeichnet, dass** eine adhäsive Lage (47) die erste absorbierende Lage (46) mit der zweiten absorbierenden Lage (48) verbindet, und dass es einen freien Raum in der adhäsiven Lage (47) im zweiten Abschnitt (72) der zentralen absorbierenden Zone (76) dergestalt gibt, dass die erste und die zweite absorbierende Lage (46, 48) im zweiten Abschnitt (72) nicht befestigt sind.

2. Damenbinde (20) nach Anspruch 1, **dadurch gekennzeichnet, dass** die zentrale absorbierende Zone (76) einen zentral befindlichen, sich longitudinal erstreckenden ersten Abschnitt (74) und einen zweiten Abschnitt (72) angrenzend an jeden longitudinalen Rand der zentralen absorbierenden Zone (76) umfasst.

3. Damenbinde (20), wie in Anspruch 1 beansprucht, **dadurch gekennzeichnet, dass** die Steifigkeit des ersten Abschnitts (74) mehr als 300 Gramm beträgt, wie gemäß dem Spitzenbiegungssteifigkeitsverfahren gemessen.

4. Damenbinde (20), wie in Anspruch 1 beansprucht, **dadurch gekennzeichnet, dass** die Steifigkeit des ersten Abschnitts (74) mehr als 350 mg beträgt, wie gemäß dem Gurley Steifigkeitsverfahren gemessen.

5. Damenbinde (20), wie in Anspruch 3 beansprucht, **dadurch gekennzeichnet, dass** der zweite Abschnitt (72) eine Steifigkeit aufweist, die geringer als 80 % der Steifigkeit des ersten Abschnitts (74) ist.

6. Damenbinde (20), wie in Anspruch 1 beansprucht, **dadurch gekennzeichnet, dass** der zweite Abschnitt (72) eine Steifigkeit aufweist, die geringer als 60 % der Steifigkeit des ersten Abschnitts (74) ist.

## Revendications

1. Serviette hygiénique (20) ayant une épaisseur inférieure à 5 mm, et comportant une zone absorbante centrale (76) ; la zone absorbante centrale (76) comportant une couche de recouvrement en vis-à-vis du corps, perméable à un fluide (42) ; une couche barrière en vis-à-vis d'un sous-vêtement, imperméable un fluide (50), et un système absorbant (44) ; la concentration en matériaux absorbants étant uniforme dans la zone absorbante centrale (76) ; la zone absorbante centrale (76) ayant une largeur d'au moins 45 mm ; la zone absorbante centrale (76) comportant en outre une première zone (74) et une seconde zone (72) adjacente à la première zone (74), la première zone (74) ayant une rigidité, et la seconde zone (72) ayant une rigidité réduite qui est inférieure à 90 % de la rigidité de la première zone (74) ; le système absorbant (44) ayant des première (46) et seconde (48) couches absorbantes; la seconde zone (72) comportant des entailles dans la première (46) ou dans la seconde couche absorbante (48), ou dans les deux, **caractérisée en ce que** une couche adhésive (47) fixe la première couche absorbante (46) sur la seconde couche absorbante (48), et **en ce qu'**il y a un vide dans la couche adhésive (47) dans la seconde zone (72) de la zone absorbante centrale (68), de sorte que les première et seconde couches absorbantes (46,48) ne sont pas fixées dans ladite seconde zone (72).

2. Serviette hygiénique (20) selon la revendication 1, dans laquelle la zone absorbante centrale (76) comporte une première zone s'étendant longitudinalement positionnée centralement (74), et une seconde zone (72) adjacente à chaque bord longitudinal de la zone absorbante centrale (76).

3. Serviette hygiénique (20) selon la revendication 1, dans laquelle ladite rigidité de la première zone (74) est supérieure à 300 grammes lorsque mesurée par le procédé de rigidité maximum en flexion.

4. Serviette hygiénique (20) selon la revendication 1, dans laquelle ladite rigidité de la première zone (74) est supérieure à 350 mg lorsque mesurée par le procédé de rigidité de Gurley.

5. Serviette hygiénique (20) selon la revendication 3, dans laquelle ladite seconde zone (72) a une rigidité qui est inférieure à 80 % de la rigidité de la première zone (74).

6. Serviette hygiénique selon la revendication 1, dans laquelle ladite seconde zone (72) a une rigidité qui est inférieure à 60 % de la rigidité de la première zone (74).
